# EUROPEAN PATENT SPECIFICATION

(11) **EP 3 072 531 B1**
(45) Date of publication and mention of the grant of the patent: **09.01.2019**
(21) Application number: 16162441.6
(22) Date of filing: 24.03.2016
(51) Int. Cl.: A61L 2/18, A47L 15/00, B08B 3/02

(54) **MACHINE FOR WASHING AND/OR THERMAL-DISINFECTION AND/OR STERILIZATION OF OBJECTS**
VORRICHTUNG ZUM WASCHEN UND/ODER THERMISCHE DESINFEKTION UND/ODER UND/ODER STERILISATION VON GEGENSTÄNDEN
MACHINE POUR LE LAVAGE ET/OU LA DESINFECTION THERMIQUE ET/OU LA STERILISATION D'OBJETS

(30) Priority: 26.03.2015 IT UD20150040
(43) Date of publication of application: 28.09.2016
(73) Proprietor: Steelco S.p.A., 31039 Riese Pio X (IT)
(72) Inventor: Zardini, Fabio, 31033 Castelfranco Veneto (TV) (IT); Casonato, Ottorino, 31033 Castelfranco Veneto (TV) (IT); Capovilla, Ivone, 31037 Loria (TV) (IT)
(74) Representative: Petraz, Davide Luigi

(56) References cited:
- EP-A1- 0 559 466

## Description

### FIELD OF THE INVENTION

Embodiments described here concern a machine for the treatment of objects used for sanitation, medical or veterinary use, for example devices for containing laboratory animals, surgical instruments, laboratory instruments or sanitation furnishing elements, for example in hospitals, such as hospital beds or trolleys, using a treatment liquid, in particular for the washing and/or thermal-disinfection and/or sterilization of said objects.

### BACKGROUND OF THE INVENTION

In the field of treating objects used in the medical or veterinary sector, or any other where it is necessary to wash and/or heat-disinfect and/or sterilize objects used in the sanitation or medical field or in research laboratories, such as for example devices for containing laboratory animals, instruments for the operating theater, laboratory instruments, hospital furnishings, for example hospital beds or trolleys, it is known to use specific treatment apparatuses, where treatment includes washing and/or thermal-disinfection and/or sterilization. These known apparatuses are provided with a treatment chamber inside which the objects to be treated are introduced on each occasion, typically supported by trolleyed transport means. Inside the treatment chamber, elements are provided to distribute the treatment fluid, for example liquids but also steam in some cases, to carry out a desired treatment cycle.

To this purpose, a hydraulic distribution unit of the treatment fluid is provided, with a pump and tubes that carry the treatment fluid inside the treatment chamber. The treatment fluid can come from various sources, for example the washing liquid can come from collection tanks housed vertically inside a technical compartment adjacent to the treatment chamber, but separate from it.

The vertically disposed collection tanks have an oblong shape and with their main axis of development disposed perpendicular to the bottom wall of the technical compartment. The technical compartments are often very limited in size, to reduce bulk, and therefore they are narrow spaces in which an operator can move, but with difficulty. The technical compartments typically have a front access door, usually adjacent to the main access door to the treatment chamber.

One disadvantage is that, during programmed or extraordinary maintenance interventions, where access to the technical compartment is required for the authorized personnel, this operation is not easy or quick because of the large bulk of the vertically disposed tanks, which in practice largely obstruct the passage through the front access door of the technical compartment, and because of the reduced space inside the technical compartment.

Document EP-A-0.559.466 describes a known dishwasher machine.

There is therefore a need to perfect a treatment machine that can overcome at least one of the disadvantages of the state of the art.

In particular, the purpose of the present invention is to reduce the bulk of the collection tanks inside the technical compartment.

The Applicant has devised, tested and embodied the present invention to overcome the shortcomings of the state of the art and to obtain these and other purposes and advantages.

Other limitations and disadvantages of conventional solutions and technologies will be clear to a person of skill after reading the remaining part of the present description with reference to the drawings and the description of the embodiments that follow, although it is clear that the description of the state of the art connected to the present description must not be considered an admission that what is described here is already known from the state of the prior art.

### SUMMARY OF THE INVENTION

The present invention is set forth and characterized in the independent claim, while the dependent claims describe other characteristics of the invention or variants to the main inventive idea.

Embodiments described here concern a machine for the treatment of objects, in particular for washing and/or thermal-disinfection and/or sterilization.

According to the invention, the treatment machine includes:
- a treatment chamber delimited by two lateral walls, a front wall, a rear wall, a bottom wall and an upper wall;
- a technical compartment adjacent to the treatment chamber, and delimited by a front wall, an upper wall, a rear wall, a bottom wall, an external lateral wall and an internal lateral wall which is a lateral and/or rear wall at least partly in common with said treatment chamber.

According to the invention, inside the technical compartment, there are one or more tanks for collecting liquid, oblong in shape and with a longitudinal main axis of development.

Moreover, the machine for treating objects comprises:
- a hydraulic distribution unit configured to distribute the treatment liquid from the one or more liquid collection tanks to the treatment chamber;
- one or more devices to move liquid disposed in the technical compartment and associated with the one or more liquid collection tanks and with the hydraulic distribution unit.

According to the invention, the one or more liquid collection tanks are disposed in the technical compartment so that a respective longitudinal main axis of development of each of the one or more liquid collection tanks is parallel to the bottom wall of the technical compartment.

In some embodiments according to the present description, the one or more liquid collection tanks are also disposed raised with respect to the bottom wall to define at least a first free space between the liquid collection tank disposed nearest to said bottom wall and the bottom wall. The first free space comprises at least one liquid movement device of the one or more liquid movement devices, so that said at least one liquid movement device comprised by the first free space is accessible trough the technical compartment and through an entrance provided at least in the front wall and/or the rear wall of the technical compartment.

Advantageously, the first free space defined by the one or more tanks raised with respect to the bottom wall is easily accessible thanks to the entrance and, in its turn, allows easy access and passage by an operator or maintenance man, to intervene on the components of the machine accessible there which require maintenance, replacement, repair or other necessary interventions.

According to other possible embodiments, the one or more liquid collection tanks are disposed near the rear wall, or the front wall, to define at least a second free space in order to dispose at least one or more distribution elements of the hydraulic distribution unit. The second free space is accessible through the entrance.

Advantageously, therefore, at least the one or more distribution elements, being provided in the second free space which is also accessible through the entrance, are easily accessible by an operator or maintenance man.

According to a variant embodiment, the liquid collection tank nearest to the bottom wall, that is, the one disposed lowest, is disposed at a height from the bottom wall so as to define, in the technical compartment, said first free space that develops at the lower part, parallel to the bottom wall of the technical compartment, for all or part of the depth of the technical compartment. Advantageously, the first free space has sizes that make it easily accessible by an operator through the at least one entrance associated at least with the front wall and/or the rear wall of the technical compartment.

These and other aspects, characteristics and advantages of the present disclosure will be better understood with reference to the following description, drawings and attached claims. The drawings, which are integrated and form part of the present description, show some embodiments of the present invention, and together with the description, are intended to describe the principles of the disclosure.

The various aspects and characteristics described in the present description can be applied individually where possible. These individual aspects, for example aspects and characteristics described in the attached dependent claims, can be the object of divisional applications.

It is understood that any aspect or characteristic that is discovered, during the patenting process, to be already known, shall not be claimed and shall be the object of a disclaimer.

### BRIEF DESCRIPTION OF THE DRAWINGS

These and other characteristics of the present invention will become apparent from the following description of some embodiments, given as a non-restrictive example with reference to the attached drawings wherein:
- fig. 1 is a perspective view of a treatment machine according to embodiments described here;
- fig. 2 is a lateral section of a treatment machine according to embodiments described here;
- fig. 3 is a schematic representation of a hydraulic distribution unit for the treatment liquid according to embodiments described here;
- fig. 4 is a front section of a treatment machine according to embodiments described here.

To facilitate comprehension, the same reference numbers have been used, where possible, to identify identical common elements in the drawings. It is understood that elements and characteristics of one embodiment can conveniently be incorporated into other embodiments without further clarifications.

### DETAILED DESCRIPTION OF SOME EMBODIMENTS

We shall now refer in detail to the various embodiments of the present invention, of which one or more examples are shown in the attached drawings. Each example is supplied by way of illustration of the invention and shall not be understood as a limitation thereof. For example, the characteristics shown or described insomuch as they are part of one embodiment can be adopted on, or in association with, other embodiments to produce another embodiment. It is understood that the present invention shall include all such modifications and variants.

Before describing these embodiments, we must also clarify that the present description is not limited in its application to details of the construction and disposition of the components as described in the following description using the attached drawings. The present description can provide other embodiments and can be obtained or executed in various other ways. We must also clarify that the phraseology and terminology used here is for the purposes of description only, and cannot be considered as limitative.

Embodiments described here concern a machine 10 for the treatment, in particular for washing and/or thermal-disinfection and/or sterilization, of objects, using a treatment fluid, typically a liquid. The term treatment in association with the embodiments described here can comprise the steps of prewashing in cold and/or hot water, washing in hot water, advantageously with detergents, a combination of these washes, rinsing with hot or cold water, possible sterilization or thermal disinfection, and a possible final drying.

In particular, one object that can be subjected to a washing and/or thermal-disinfection and/or sterilization treatment using the machine 10 according to embodiments described here can be for example an object used in the sanitation, medical or veterinary field, for example a device for containing laboratory animals, surgical instruments, laboratory instruments or sanitation furnishing elements, for example in hospitals, such as hospital beds or trolleys. Hereafter, by way of example, with regard to objects to be treated by the machine 10, we will refer to containing devices 12 for veterinary use, for example cages for animals.

Hereafter, treatment liquid can mean water without detergents, or solutions of water with detergents or chemical additives in general.

In a particular embodiment, the machine 10 can be a specific operating unit in which one or more treatment steps can be carried out.

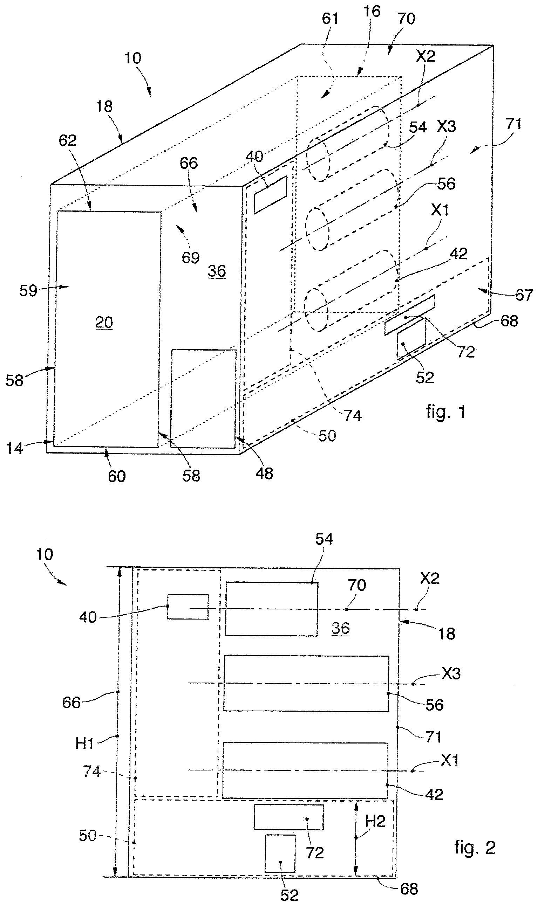

In one embodiment, the machine 10 can be disposed aligned with one or more operating units along an imaginary operating axis, according to a configuration known as "pass-through". In this case described using fig. 1 for example, the machine 10 can comprise a loading aperture 14.

In a position opposite the loading aperture 14, an unloading aperture 16 is disposed, also comprised in the machine 10.

In another embodiment, the machine 10 can be disposed in a path of parallel branches, adjacent, in which one of the ends of one branch is connected to the end of the other branch and the other ends of the branches respectively define a loading end and an unloading end of the containing devices 12. In this case, a single-side configuration of the first machine 10 is defined, that is, the loading and unloading apertures are disposed in correspondence with the same operating side.

The machine 10 can comprise a treatment chamber 20.

The treatment chamber 20 can be delimited by two lateral walls 58, a front wall 59, a rear wall 61, a bottom wall 60 and an upper wall 62.

The treatment chamber 20 can have, for example, a parallelepiped shape with a quadrangular, rectangular or square base.

The machine 10 can comprise a technical compartment 36, adjacent to the treatment chamber 20.

The technical compartment 36 can be delimited by a front wall 66, an upper wall 70, a rear wall 71, a bottom wall 68, an external lateral wall 67 and an internal lateral wall 69.

The internal lateral wall 69 can be a lateral 58 and/or rear wall 61 at least partly or completely in common with the treatment chamber 20.

In a particular embodiment, the treatment chamber 20 and the technical compartment 36 can be defined by containing bodies that are separate and contiguous and/or communicating with each other.

In another embodiment, the front wall 66, the upper wall 70, the rear wall 71, the bottom wall 68 and the external lateral wall 67 can be defined by, or be part of, an external containing body 18.

The external containing body 18 can have a bigger volume than the treatment chamber 20 so as to be able to comprise the treatment chamber 20 inside it.

The external containing body 18 can have the function of protecting, enclosing and containing the machine 10.

The external containing body 18 can have a parallelepiped shape with a quadrangular, rectangular or square base.

For example, without wishing to limit the field of protection of the present invention, the external containing body 18 can be configured to have a width from 2 m to 3 m, a height from 2 m to 3.5 m and a depth from 2 m to 3.5 m. the height of the external containing body 18 is defined as H₁.

In some embodiments, the technical compartment 36 can be provided with at least one entrance 48 provided at least in the front wall 66 and/or the rear wall 71 of the technical compartment 36.

Inside the technical compartment 36 there can be one or more tanks 42, 54, 56 for collecting liquid, oblong in shape and having respectively a longitudinal main axis of development X₁, X₂, X₃.

The one or more liquid collection tanks 42, 54, 56 can be disposed in the technical compartment 36 so that a respective longitudinal main axis of development X₁, X₂, X₃ of each of the one or more liquid collection tanks 42, 54, 56 is parallel to the bottom wall 68. Advantageously, this position of the one or more liquid collection tanks 42, 54, 56 allows to prevent the stratification inside them of the treatment liquid due to the different temperature. For example, the main axes of development X₁, X₂, X₃ of each of the one or more liquid collection tanks 42, 54, 56 can be horizontal.

Moreover, the one or more liquid collection tanks 42, 54, 56 can be disposed raised with respect to the bottom wall 68 of the technical compartment 36.

In particular, the liquid collection tank 42 of the one or more liquid collection tanks 42, 54, 56 can be disposed nearest to the bottom wall 68 at a height H₂, in other words it is the one disposed lowest.

For example, without wishing to limit the field of protection of the present invention, the ratio between height H₂ and height H₁ can be comprised between 0.15 and 0.35, in particular between 0.2 and 0.3.

The liquid collection tank 42, thus disposed, defines in the technical compartment 36 a first free space 50, or first free maneuvering space, having essentially a height H₂ that develops below and parallel to the bottom wall 68.

The first free space 50 can develop for all or part of the depth of the technical compartment 36.

The first free space 50 can have sizes that make it easily accessible for an operator through at least the entrance 48.

Therefore, the one or more liquid collection tanks 42, 54, 56 are disposed raised with respect to the bottom wall 68 to define at least the first free space 50 between the liquid collection tank 42 disposed nearest to the bottom wall 68 and the bottom wall 68. Thanks to the entrance 48, the first free space 50 is easily accessible from the outside by an operator or maintenance man who, in his turn, can enter the first free space to intervene on the components, for example indicated by the reference numbers 52, 72 hereafter in the description, of the machine 10 accessible there which require maintenance, replacement, repairs or other necessary interventions.

This technical solution allows to prevent long stoppages of the plant and hence to reduce the machine downtimes caused, in the state of the art, by the bulk of the vertically disposed tanks, that is, with their axis perpendicular to the bottom wall, inside the technical compartment 36.

In a particular embodiment, the one or more liquid collection tanks 42, 54, 56 can be installed so as to be positioned near the rear wall 71 or the front wall 66.

This disposition of the one or more liquid collection tanks 42, 54, 56 comprised in the technical compartment 36 allows to create, at the front or rear, at least one second free space 74, or second free maneuvering space.

The second free space 74 develops from the top downward transversely, in particular perpendicularly, to the bottom wall 68.

The machine 10 also comprises a hydraulic distribution unit 38 configured to distribute the treatment liquid from the one or more liquid collection tanks 42, 54, 56 to the treatment chamber 20.

Furthermore, the machine 10 comprises at least a liquid movement device 40, 52, 72 disposed in the technical compartment 36 and associated with the one or more liquid collection tanks 42, 54, 56 and the hydraulic distribution unit 38. For example, as described in detail hereafter, liquid movement devices 52, 72 can be provided associated with the first free space 50 and possibly one liquid movement device 40, associated with the second free space 74.

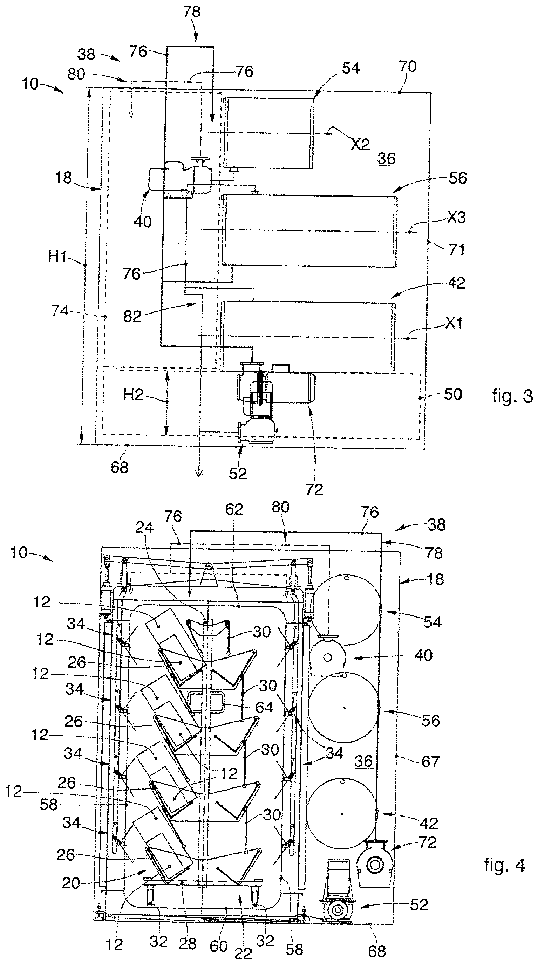

The second free space 74 is useful for installing at least the distribution elements 76 comprised in or associated with the hydraulic distribution unit 38 in a single small space (fig. 3).

By distribution elements usable in association with the embodiments described here we mean at least the liquid movement device 40, 52, 72 and at least one, or a plurality of, said distribution elements 76 (figs. 2 and 3).

Merely by way of example, liquid movement device 40, 52, 72 can mean a pump driven by a drive member to allow to transfer the treatment liquid from the liquid collection tanks 42, 54, 56 to the treatment chamber 20.

Therefore, the second free space 74 can be provided to dispose at least one or more distribution elements 76 of the hydraulic distribution unit 38. Advantageously, therefore, at least the one or more distribution elements 76, being provided in the second free space 74 which is also accessible though the entrance 48, are easily accessible by an operator or maintenance man.

The hydraulic distribution unit 38 is made to transfer, by means of distribution elements 76 connected with each other, the treatment liquid taken from the first liquid collection tank 42, or the second liquid collection tank 54, or the third liquid collection tank 56, according to the step performed inside the treatment chamber 20.

In a particular embodiment, the second free space 74 can comprise a plurality of distribution elements 76 and a liquid movement device 40. The latter are therefore easily accessible through the entrance 48 from the second free space 74.

In a particular embodiment, the entrance 48 can be associated at least with the front wall 66 and/or the rear wall 71 of the technical compartment 36.

By entrance 48 "associated" at least with the front wall 66, we mean that the entrance 48 is made in correspondence with the front wall 66, or made in correspondence with the external lateral wall 67 close to the point where it connects to the front wall 66. In both cases the entrance 48 can allow access inside the technical compartment 36 to an operator in correspondence with the first free space 50 and in the condition where one or more liquid collection tanks 42, 54, 56 are close to the rear wall 71.

By entrance 48 "associated" at least with the rear wall 71, we mean that the entrance 48 is made in correspondence with the rear wall 71, or made in correspondence with the external lateral wall 67 close to the point where it connects to the rear wall 71. In both cases the entrance 48 can allow access inside the technical compartment 36 to an operator in correspondence with the first free space 50 and in the condition where one or more liquid collection tanks 42, 54, 56 are close to the front wall 66.

In a particular embodiment shown in figs. 1-4, inside the technical compartment 36, a first liquid collection tank 42 can be disposed, able to collect a treatment liquid, for example an alkaline or acid solution, for the washing step. The liquid collection tank 42 is configured and installed in the embodiment described above.

Furthermore, inside the technical compartment 36 at least another second liquid collection tank 54 can be disposed, to collect a treatment liquid, for example water, to carry out the rinsing step.

The second liquid collection tank 54 can be positioned in a position immediately above the first liquid collection tank 42.

In another embodiment, the technical compartment 36 can comprise at least a third liquid collection tank 56.

The third collection tank 56 collects a treatment liquid, for example an alkaline or acid solution, to carry out another washing step in addition to that performed by taking the treatment liquid from the first liquid collection tank 42.

In possible examples, the one or more liquid collection tanks 42, 54, 56 can have a volume such as to be able to contain from 110 L to 250 L of treatment liquid.

In particular, the liquid collection tanks 42, 56 for performing a washing step can have a volume from 200 L to 250 L and the liquid collection tank 54 for performing the rinsing step can have a volume from 110 L to 150 L.

In a variant embodiment shown in figs. 1-4, the first free space 50 can comprise two more liquid movement devices 52, 72. These can therefore be present or installed in the first free space 50 and, since the tanks 42, 54, 56 are raised with respect to the bottom wall 68, they are easily accessible through the entrance 48 from the first free space 50.

The hydraulic distribution unit 38 can comprise a washing circuit 78 indicated by a continuous bold line (as shown in figs. 3 and 4).

The washing circuit 78 can be connected to the first liquid collection tank 42 and/or the third liquid collection tank 56.

The washing circuit 78 takes the treatment liquid, that is, an acid and or alkaline solution, from the first liquid collection tank 42 and/or the third liquid collection tank 56, so that it can introduce it into the treatment chamber 20.

The treatment liquid is distributed by the distribution elements 76 and the at least one liquid movement device 72.

The hydraulic distribution unit 38 can comprise a rinsing circuit 80 indicated by a line of dashes (as shown in figs. 3 and 4).

The rinsing circuit 80 can be connected to the second liquid collection tank 54.

The rinsing circuit 80 takes the treatment liquid, used for rinsing, from the second liquid collection tank 54, so that it can introduce it into the treatment chamber 20.

The treatment liquid is distributed from the second liquid collection tank 54 to the treatment chamber 20 by the distribution elements 76 and the liquid movement device 40.

In a particular embodiment, the hydraulic distribution unit 38 can comprise a recirculating circuit 82, indicated by a thin continuous line (as shown in fig. 3).

The recirculating circuit 82 can be configured to take the treatment liquid coming from the treatment chamber 20 and re-use it for subsequent treatment steps.

Therefore, from the treatment chamber 20 the treatment liquid can be reintroduced inside the liquid collection tanks 42, 56.

The recirculating circuit 82 provides to transport the treatment liquid through the distribution elements 76 and the liquid movement device 52.

The treatment chamber 20, as described using fig. 4, can comprise at least one support trolley 22.

The support trolley 22 can comprise a support frame 24 to support one or more containing devices 12.

The support frame 24 can comprise one or more support planes 26 advantageously inclined to allow, for example, the treatment liquid used to flow away.

According to a possible embodiment, the support planes 26 can be disposed one on top of the other to define at least a column of support planes 26.

According to another embodiment, the support planes 26 can be disposed parallel and adjacent to each other. In this case the support planes 26 can define two or more columns located adjacent to each other.

Each support plane 26, except for the support plane 26 most adjacent to base 28 of the support frame 24, comprises a positioning element 30 to keep the containing device 12 stationary and in its seating in correspondence with the support plane 26.

According to a possible solution, the support planes 26 are inclined by an angle of about 45° with respect to the horizontal.

According to a possible solution, the support trolley 22 is selectively movable on wheels 32 by the thrust action of a feed device, for example a cable, or a belt or a chain.

The treatment chamber 20 can comprise one or more nozzles 34 to deliver the treatment liquid inside the treatment chamber 20. The nozzles 34 are installed so that they are distributed along the height of one or more lateral walls 58 of the treatment chamber 20.

The nozzles 34 can be fed by a hydraulic distribution unit 38.

In a particular embodiment, the number of nozzles 34 can correspond to the number of support planes 26 and can be installed in correspondence with each support plane 26.

In a particular embodiment, the nozzles 34 can be disposed on at least one lateral wall 58 of the treatment chamber 20.

In another embodiment described using fig. 4, the nozzles 34 can be disposed in correspondence with the two lateral walls 58 of the treatment chamber 20 and opposite each other.

In another embodiment, the nozzles 34 can be disposed in correspondence with the upper wall 62 of the treatment chamber 20.

In another embodiment, the nozzles 34 can be disposed in correspondence with one or more lateral walls 58 and with the upper wall 62.

In another embodiment, not shown in the drawings and combinable with the previous embodiments, the support trolley 22 can comprise one or more rotors 64 installed in correspondence with the support frame 24. Each rotor 64 is provided with nozzles to deliver the treatment liquid and is configured so as to rotate upon itself when the machine 10 is functioning.

It is clear that modifications and/or additions of parts may be made to the machine 10 as described heretofore, without departing from the field and scope of the present invention.

In the following claims, the sole purpose of the references in brackets is to facilitate reading: they must not be considered as restrictive factors with regard to the field of protection claimed in the specific claims.

## Claims

1. Machine for the treatment of washing, thermal-disinfection and/or sterilization of objects (12), said machine comprising:
- a treatment chamber (20) delimited by two lateral walls (58), a front wall (59), a rear wall (61), a bottom wall (60) and an upper wall (62);
- a technical compartment (36) adjacent to the treatment chamber (20) and delimited by a front wall (66), an upper wall (70), a rear wall (71), a bottom wall (68), an external lateral wall (67) and an internal lateral wall (69) which is a lateral (58) and/or rear (61) wall at least partly in common with said treatment chamber (20),
there being, inside the technical compartment (36), one or more tanks (42, 54, 56) for collecting liquid, oblong in shape and with a longitudinal main axis of development (X₁, X₂, X₃),
**characterized in that** said machine comprises:
- a hydraulic distribution unit (38) configured to distribute the treatment liquid from said one or more liquid collection tanks (42, 54, 56) to said treatment chamber (20);
- one or more devices to move liquid (40, 52, 72) disposed in the technical compartment (36) and associated with said one or more liquid collection tanks (42, 54, 56) and with the hydraulic distribution unit (38);
wherein said one or more liquid collection tanks (42, 54, 56) are disposed in the technical compartment (36) so that a respective longitudinal main axis of development (X₁, X₂, X₃) of each of said one or more liquid collection tanks (42, 54, 56) is parallel to said bottom wall (68) of the technical compartment (36); wherein said one or more liquid collection tanks (42, 54, 56) are disposed raised with respect to said bottom wall (68) to define at least a first free space (50) between the liquid collection tank (42) disposed nearest to said bottom wall (68), and wherein said first free space (50) comprises at least one liquid movement device (52, 72) of said one or more liquid movement devices (40, 52, 72), so that said at least one liquid movement device (52, 72) comprised by said first free space (50) is accessible through said technical compartment (36) and through an entrance (48) provided at least in the front wall (66) and/or the rear wall (71) of said technical compartment (36).

2. Machine as in claim 1, **characterized in that** the liquid collection tank (42) nearest to said bottom wall (68) is disposed at a height (H₂) from said bottom wall (68) so as to define, in the technical compartment (36), said first free space (50) that develops at the lower part, parallel to said bottom wall (68) of the technical compartment (36), for all or part of the depth of said technical compartment (36).

3. Machine as in claim 1 or 2, **characterized in that** the technical compartment (36) comprises, as well as said first liquid collection tank (42) to collect said treatment liquid used in a washing step inside the treatment chamber (20), also a second liquid collection tank (54) to collect said treatment liquid used in a rinsing step inside said treatment chamber (20), **and in that** said technical compartment (36) comprises at least a third liquid collection tank (56) to collect said treatment liquid to carry out another washing step inside the treatment chamber (20).

4. Machine as in claim 1, 2 or 3, **characterized in that** said one or more liquid collection tanks (42, 54, 56) are disposed near the rear wall (71), or the front wall (66), to define at least a second free space (74) in order to dispose at least one or more distribution elements (76) of said hydraulic distribution unit (38), said second free space (74) being accessible through said entrance (48).

5. Machine as in claim 4, **characterized in that** said entrance (48) is at the front and made on a front wall (66) of the technical compartment (36) and said one or more liquid collection tanks (42, 54, 56) are disposed near the rear wall (71) and so as to define at the front said second free space (74) that develops transversely to said bottom wall (68), in correspondence to said entrance (48).

6. Machine as in claim 5, **characterized in that** said distribution elements (76) associated with said hydraulic distribution unit (38) and liquid movement device (40) are present and accessible in said second free space (74).

7. Machine as in any of the claims from 1 to 6, **characterized in that** said treatment chamber (20) is configured pass-through, comprising a loading aperture (14) and an unloading aperture (16) disposed in an opposite position with respect to each other.

8. Machine as in any of the claims from 1 to 6, **characterized in that** said treatment chamber (20) is configured single-side, comprising a single aperture that serves for loading or unloading.

9. Machine as in any of the claims from 1 to 8, **characterized in that** said front wall (66), upper wall (70), rear wall (71), bottom wall (68) and external lateral wall (67) are defined by an external containing body (18) with a height (H₁) and volume greater than the treatment chamber (20) and said external containing body (18) comprising said treatment chamber (20).

10. Machine as in claim 9, **characterized in that** the ratio between the height (H₂) of the first free space (50) and the height (H₁) of the external containing body (18) can be comprised between 0.15 and 0.35, in particular between 0.2 and 0.3.

11. Machine as in any of the claims from 1 to 10, **characterized in that** said one or more liquid collection tanks (42, 54, 56) are disposed with the longitudinal main axes of development (X₁, X₂, X₃) horizontal.

## Patentansprüche

1. Maschine zur Wasch-, thermische-Desinfektion- und/ oder Sterilisierungs-Behandlung von Gegenständen (12), wobei die Maschine aufweist:
- eine Behandlungskammer (20), die durch zwei Seitenwände (58), eine Vorderwand (59), eine Rückwand (61), eine untere Wand (60) und eine obere Wand (62) begrenzt ist,
- eine technische Kammer (36), die zu der Behandlungskammer (20) benachbart und durch eine Vorderwand (66), eine obere Wand (70), eine Rückwand (71), eine untere Wand (68), eine äußere Seitenwand (67) und eine innere Seitenwand (69), die eine Seiten- (58) und/ oder Rückwand (61) ist, die zumindest teilweise mit der Behandlungskammer (20) gemeinsam ist, begrenzt ist,
wobei innerhalb der technischen Kammer (36) ein oder mehrere Behälter (42, 54, 56) zum Sammeln von Flüssigkeit vorhanden sind, die eine längliche Form haben und mit einer längsverlaufenden Hauptentwicklungsachse (X₁, X₂, X₃),
**dadurch gekennzeichnet, dass** die Maschine aufweist:
- eine hydraulische Verteilereinheit (38), die konfiguriert ist, um die Behandlungsflüssigkeit aus dem einen oder den mehreren Flüssigkeitssammelbehältern (42, 54, 56) an die Behandlungskammer (20) zu verteilen,
- eine oder mehrere Vorrichtungen zum Bewegen von Flüssigkeit (40, 52, 72), die in der technischen Kammer (36) angeordnet und mit dem einen oder den mehreren Flüssigkeitssammelbehältern (42, 54, 56) und mit der hydraulischen Verteilereinheit (38) verbunden sind,
wobei der eine oder die mehreren Flüssigkeitssammelbehälter (42, 54, 56) in der technischen Kammer (36) angeordnet sind, so dass eine jeweilige längsverlaufende Hauptentwicklungsachse (X₁, X₂, X₃) von jedem von dem einen oder den mehreren Flüssigkeitssammelbehältern (42, 54, 56) parallel zu der unteren Wand (68) der technischen Kammer (36) ist,
wobei der eine oder die mehreren Flüssigkeitssammelbehälter (42, 54, 56) bezüglich der unteren Wand (68) erhöht angeordnet sind, um mindestens einen ersten freien Raum (50) zwischen dem Flüssigkeitssammelbehälter (42), der am nächsten bei der unteren Wand (68) angeordnet ist, zu definieren,
und wobei der erste freie Raum (50) mindestens eine Flüssigkeitsbewegungsvorrichtung (52, 72) von der einen oder den mehreren Flüssigkeitsbewegungsvorrichtungen (40, 52, 72) aufweist, so dass die mindestens eine Flüssigkeitsbewegungsvorrichtung (52, 72), die in dem ersten freien Raum (50) enthalten ist, durch die technische Kammer (36) und durch einen Einlass (48) hindurch zugänglich ist, der mindestens in der Vorderwand (66) und/oder der Rückwand (71) der technischen Kammer (36) vorgesehen ist.

2. Maschine wie in Anspruch 1, **dadurch gekennzeichnet, dass** der Flüssigkeitssammelbehälter (42), der am nächsten bei der unteren Wand (68) liegt, auf einer Höhe (H₂) von der unteren Wand (68) aus angeordnet ist, um in der technischen Kammer (36) den ersten freien Raum (50) zu definieren, der sich am unteren Teil parallel zu der unteren Wand (68) der technischen Kammer (36) über die Gesamtheit oder einen Teil der Tiefe der technischen Kammer (36) entwickelt.

3. Maschine wie in Anspruch 1 oder 2, **dadurch gekennzeichnet, dass** die technische Kammer (36) sowohl den ersten Flüssigkeitssammelbehälter (42) zum Sammeln der Behandlungsflüssigkeit, die in einem Waschschritt innerhalb der Behandlungskammer (20) verwendet wird, als auch einen zweiten Flüssigkeitssammelbehälter (54) aufweist, um die Behandlungsflüssigkeit, die während eines Spülschrittes innerhalb der Behandlungskammer (20) verwendet wird, zu sammeln, und dass die technische Kammer (36) mindestens einen dritten Flüssigkeitssammelbehälter (56) zum Sammeln der Behandlungsflüssigkeit aufweist, um einen weiteren Waschschritt innerhalb der Behandlungskammer (20) durchzuführen.

4. Maschine wie in Anspruch 1, 2 oder 3, **dadurch gekennzeichnet, dass** der eine oder die mehreren Flüssigkeitssammelbehälter (42, 54, 56) nahe bei der Rückwand (71) oder der Vorderwand (66) angeordnet sind, um mindestens einen zweiten freien Raum (74) zu definieren, um mindestens einen oder mehrere Verteilerelemente (76) der hydraulischen Verteilereinheit (38) anzuordnen, wobei der zweite freie Raum (74) durch den Einlass (48) hindurch zugänglich ist.

5. Maschine wie in Anspruch 4, **dadurch gekennzeichnet, dass** der Einlass (48) an der Vorderseite ist und an einer Vorderwand (66) der technischen Kammer (36) ausgebildet ist und der eine oder die mehreren Flüssigkeitssammelbehälter (42, 54, 56) nahe bei der Rückwand (71) und derart angeordnet sind, um an der Vorderseite den zweiten freien Raum (74) zu definieren, der sich quer zu der unteren Wand (68) mit dem Einlass (48) korrespondierend entwickelt.

6. Maschine wie in Anspruch 5, **dadurch gekennzeichnet, dass** die Verteilerelemente (76), die mit der hydraulischen Verteilereinheit (38) und der Flüssigkeitsbewegungsvorrichtung (40) verknüpft sind, in dem zweiten freien Raum (74) vorliegen und zugänglich sind.

7. Maschine wie in irgendeinem der Ansprüche 1 bis 6, **dadurch gekennzeichnet, dass** die Behandlungskammer (20) hindurchführend konfiguriert ist, wobei sie eine Beladeöffnung (14) und eine Entladeöffnung (16) aufweist, die bezüglich einander in einer entgegengesetzten Position angeordnet sind.

8. Maschine wie in irgendeinem der Ansprüche 1 bis 6, **dadurch gekennzeichnet, dass** die Behandlungskammer (20) einseitig konfiguriert ist, wobei sie eine einzige Öffnung aufweist, die dem Laden oder Entladen dient.

9. Maschine wie in irgendeinem der Ansprüche 1 bis 8, **dadurch gekennzeichnet, dass** die Vorderwand (66), die obere Wand (70), die Rückwand (71), die untere Wand (68) und die äußere Seitenwand (67) durch einen äußeren Aufnahmekörper (18) definiert sind, mit einer Höhe (H₁) und einem Volumen größer als die Behandlungskammer (20), und wobei der äußere Aufnahmekörper (18) die Behandlungskammer (20) aufweist.

10. Maschine wie in Anspruch 9, **dadurch gekennzeichnet, dass** das Verhältnis zwischen der Höhe (H₂) des ersten freien Raumes (50) und der Höhe (H₁) des äußeren Aufnahmekörpers (18) zwischen 0,15 und 0,35, insbesondere zwischen 0,2 und 0,3 liegen kann.

11. Maschine wie in irgendeinem der Ansprüche 1 bis 10, **dadurch gekennzeichnet, dass** der eine oder die mehreren Flüssigkeitssammelbehälter (42, 54, 56) mit den längsverlaufenden Hauptentwicklungsachsen (X₁, X₂, X₃) horizontal angeordnet sind.

## Revendications

1. Machine pour le traitement de lavage, désinfection thermique et/ou stérilisation d'objets (12), ladite machine comprenant :
une chambre de traitement (20) délimitée par deux parois latérales (58), une paroi avant (59), une paroi arrière (61), une paroi inférieure (60) et une paroi supérieure (62) ;
un compartiment technique (36) adjacent à la chambre de traitement (20) et délimité par une paroi avant (66), une paroi supérieure (70), une paroi arrière (71), une paroi inférieure (68), une paroi latérale externe (67) et une paroi latérale interne (69) qui est une paroi latérale (58) et/ou arrière (61) au moins partiellement commune avec ladite chambre de traitement (20),
à l'intérieur du compartiment technique (36), un ou plusieurs réservoirs (42, 54, 56) destinés à recueillir du liquide, de forme oblongue et ayant un axe de développement principal longitudinal (X₁, X₂, X₃),
**caractérisé en ce que** ladite machine comprend :
une unité de distribution hydraulique (38) configurée pour distribuer le liquide de traitement depuis lesdits un ou plusieurs réservoirs de recueil de liquide (42, 54, 56) vers ladite chambre de traitement (20) ;
un ou plusieurs dispositifs pour déplacer le liquide (40, 52, 72) disposés dans le compartiment technique (36) et associés auxdits un ou plusieurs réservoirs de recueil de liquide (42, 54, 56) et à l'unité de distribution hydraulique (38) ;
dans laquelle lesdits un ou plusieurs réservoirs de recueil de liquide (42, 54, 56) sont disposés dans le compartiment technique (36) de sorte qu'un axe de développement principal longitudinal respectif (X₁, X₂, X₃) de chacun desdits un ou plusieurs réservoirs de recueil de liquide (42, 54, 56) est parallèle à ladite paroi inférieure (68) du compartiment technique (36) ;
dans laquelle lesdits un ou plusieurs réservoirs de recueil de liquide (42, 54, 56) sont disposés surélevés par rapport à ladite paroi inférieure (68) pour définir au moins un premier espace libre (50) dans le réservoir de recueil de liquide (42) disposé le plus proche ladite paroi inférieure (68),
et dans lequel ledit premier espace libre (50) comprend au moins un dispositif de déplacement de liquide (52, 72) desdits un ou plusieurs dispositifs de déplacement de liquide (40, 52, 72), de sorte que ledit au moins un dispositif de déplacement de liquide (52, 72) constitué par ledit premier espace libre (50) est accessible via ledit compartiment technique (36) et via une entrée (48) prévue au moins dans la paroi avant (66) et / ou la paroi arrière (71) dudit compartiment technique (36).

2. Machine selon la revendication 1, **caractérisée en ce que** le réservoir de recueil de liquide (42) le plus proche de ladite paroi inférieure (68) est disposé à une hauteur (H₂) à partir de ladite paroi inférieure (68) de manière à définir, dans le compartiment technique (36), ledit premier espace libre (50) qui se développe au niveau de la partie inférieure, parallèlement à ladite paroi inférieure (68) du compartiment technique (36), pour tout ou partie de la profondeur dudit compartiment technique (36).

3. Machine selon la revendication 1 ou 2, **caractérisée en ce que** le compartiment technique (36) comprend, outre ledit premier réservoir de recueil de liquide (42) pour recueillir ledit liquide de traitement utilisé dans une étape de lavage à l'intérieur de la chambre de traitement (20), également un deuxième réservoir de recueil de liquide (54) pour recueillir ledit liquide de traitement utilisé dans une étape de rinçage à l'intérieur de ladite chambre de traitement (20), et **en ce que** ledit compartiment technique (36) comprend au moins un troisième réservoir de recueil de liquide (56) pour recueillir ledit liquide de traitement pour effectuer une autre étape de lavage à l'intérieur de la chambre de traitement (20).

4. Machine selon la revendication 1, 2 ou 3, **caractérisée en ce que** lesdits un ou plusieurs réservoirs de recueil de liquide (42, 54, 56) sont disposés à proximité de la paroi arrière (71) ou de la paroi avant (66), pour définir au moins un second espace libre (74) pour disposer au moins un ou plusieurs éléments de distribution (76) de ladite unité de distribution hydraulique (38), ledit second espace libre (74) étant accessible via ladite entrée (48).

5. Machine selon la revendication 4, **caractérisée en ce que** ladite entrée (48) se trouve à l'avant et est réalisée sur une paroi avant (66) du compartiment technique (36) et lesdits un ou plusieurs réservoirs de recueil de liquide (42, 54, 56) sont disposés à proximité de la paroi arrière (71) et de manière à définir à l'avant ledit second espace libre (74) qui se développe transversalement à ladite paroi inférieure (68), en correspondance avec ladite entrée (48).

6. Machine selon la revendication 5, **caractérisée en ce que** lesdits éléments de distribution (76) associés à ladite unité de distribution hydraulique (38) et au dispositif de déplacement de liquide (40) sont présents et accessibles dans ledit second espace libre (74).

7. Machine selon l'une quelconque des revendications 1 à 6, **caractérisée en ce que** ladite chambre de traitement (20) est configurée traversante, comprenant une ouverture de chargement (14) et une ouverture de déchargement (16) disposées dans une position opposée l'une par rapport à l'autre.

8. Machine selon l'une quelconque des revendications 1 à 6, **caractérisée en ce que** ladite chambre de traitement (20) est configurée d'un seul côté, comprenant une seule ouverture qui sert au chargement ou au déchargement.

9. Machine selon l'une quelconque des revendications 1 à 8, **caractérisée en ce que** ladite paroi avant (66), ladite paroi supérieure (70), ladite paroi arrière (71), ladite paroi inférieure (68) et ladite paroi latérale externe (67) sont définies par un corps contenant externe (18) ayant une hauteur (H₁) et un volume supérieurs à ceux de la chambre de traitement (20), et ledit corps contenant externe (18) comprenant ladite chambre de traitement (20).

10. Machine selon la revendication 9, **caractérisée en ce que** le rapport entre la hauteur (H₂) du premier espace libre (50) et la hauteur (H₁) du corps contenant extérieur (18) peut être compris entre 0,15 et 0,35, en particulier entre 0,2 et 0,3.

11. Machine selon l'une quelconque des revendications 1 à 10, **caractérisée en ce que** lesdits un ou plusieurs réservoirs de recueil de liquide (42, 54, 56) sont disposés en ayant les axes de développement principaux longitudinaux (X₁, X₂, X₃) horizontaux.
